# EUROPEAN PATENT APPLICATION

(11) **EP 3 674 392 A1**
(43) Date of publication of application: **01.07.2020**
(21) Application number: 19219429.8
(22) Date of filing: 23.12.2019
(51) Int. Cl.: C12M 1/16, C12M 1/00

(54) **PROCESS AND PLANT FOR GROWTH OF MICROORGANISMS AND USE THEREOF**

(30) Priority: 28.12.2018 IT 201800021337
(71) Applicant: Amvic S.r.l., 20122 Milano (MI) (IT)
(72) Inventor: FERRANTE, Antonio, 20122 Milano (MI) (IT); SEDINO, Francesco, 20122 Milano (MI) (IT); STORI, Alessandro, 20122 Milano (MI) (IT)
(74) Representative: Trupiano, Federica

(57) **Abstract**

The present invention belongs to the technical field of industrial biotechnologies. In particular, the present invention refers to a plant for the growth of at least one microorganism type and to a process to obtain it.

## Description

### ABSTRACT

The present invention belongs to the technical field of industrial biotechnologies. In particular, the present invention refers to a plant for the growth of at least one microorganism type and to a process to obtain it.

### STATE OF THE ART

The use of microorganisms, such as bacteria and fungi, allowed to significantly improve the quality of life of human beings. As a matter of fact, nowadays such microorganisms are used to produce antibiotics and molecules for therapeutic use, to improve crops and for the food industry. As a matter of fact, at industrial level the fermentation processes of bacteria and yeasts are used for the production of beverages, such as beer and wine, or of food such as yogurt and fermented food. In addition, such fermentation processes are requested for the production of fuel and other chemicals. This requires that a high amount of the microorganism of interest and of the substances produced by it is available in short times and with high purity. Therefore, the cultivation of such microorganisms at industrial level can not be carried out with systems of limited capacity, which require many process steps and which therefore are more prone to contaminations.

In order to overcome such problems, industrial plants capable of producing high amounts of the microorganisms of interest have been developed. Such plants are able to produce the microorganisms of interest on industrial scale and can be bioreactors or fermentors.

However, they require high production costs due to, for example, the construction of the stirring system and high processing costs, e.g., the energy required for achieving the whole process.

Due to the high interest for the industrial application in the production of microorganisms and due to the high demand of such microorganisms, there is the need to provide an industrial plant able to optimize the growth of microorganisms.

Moreover, due to high costs of both the stirring system of the plants and the already known devices and of the energy required for the mixing of the components required in such plants and in the already known devices, a plant for the growth of microorganisms which has low costs is needed.

In the same way, there is the need to provide a process for the growth of microorganisms which, as a whole, has low costs and short times, while keeping a high qualitative and quantitative level with respect to the produced microorganism.

A further drawback of the plants known to date refers to the difficulty in maintaining the sterility of the system for long times. As a matter of fact, the plants for the growth of microorganisms require to work under sterile conditions, since the presence of contaminants can damage the microbial culture. As a matter of fact, the contaminants could cause the total loss of the production batch due to non-compliance, causing a significant damage. Therefore, the stability of the microbial culture must not deteriorate, giving rise to different products with respect to the desired ones.

There is therefore the need to provide a plant suitable to the industrial application for the production of microorganisms which has low cost and guarantees production sterility and microbial culture stability.

It is in addition necessary to develop a process for the growth of microorganisms which guarantees high yields in short times, and which moreover proves to be advantageous from an economical point of view.

### OBJECTS OF THE INVENTION

Object of the present invention is to provide a plant suitable to the industrial application and capable of optimizing the growth of microorganisms.

Moreover, object of the present invention is to provide a plant for the growth of microorganisms which has low costs.

Further object of the present invention is to provide a process for the growth of microorganisms which guarantees growth sterility and high yields in short times.

Other object of the present invention is to provide a process for the growth of microorganisms which has low costs.

These and other objects are achieved by the present invention referring to a mixing and vacuum drying plant for the growth of at least one microorganism type and to a process for the same object.

### SHORT DESCRIPTION OF THE DRAWINGS

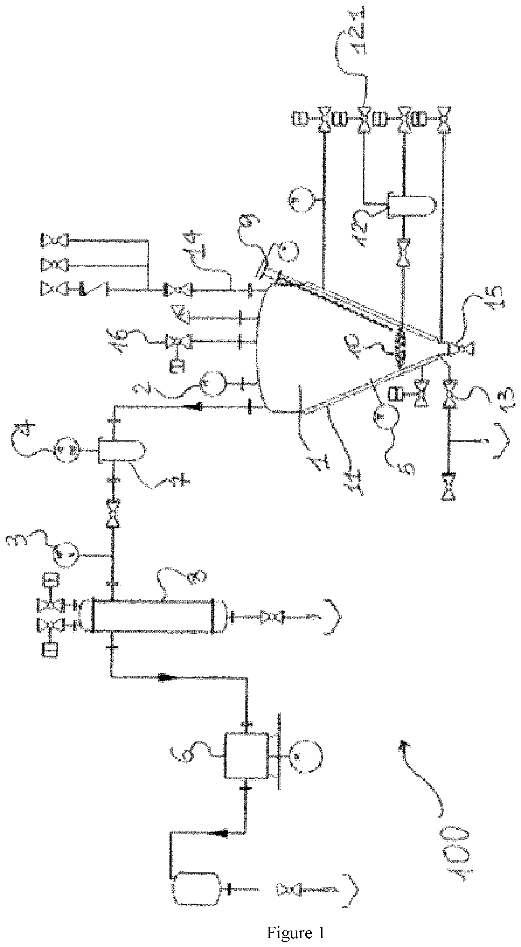

Figure 1 is a schematic representation of an implementation of the plant according to the invention.

### DESCRIPTION OF THE INVENTION

Object of the present invention is a mixing and vacuum drying plant for the growth of at least one microorganism type, comprising:
- Reactor
- Filter
- Condenser
- Vacuum pump

Advantageously, the plant of the invention allows the growth of the microorganisms and their drying in the reactor of the plant. This way, the growth of the microorganisms is coupled to the recovery of the same by drying within the same plant. Therefore, the plant of the invention allows to avoid the contamination of the microorganisms during their transport to the drying step in external equipment.

With microorganisms growth it is meant herein the numerical increase of the population of the at least one microorganism type. Therefore, such microorganisms are viable microorganisms.

The plant for the growth of microorganisms according to the present invention is a mixing and vacuum drying plant which can be used to produce cultures of viable microorganisms, viable spores of said microorganisms, enzymes and secondary metabolites. According to a preferred embodiment, the plant of the invention is used to produce cultures of microorganisms such as fungi (Trichoderma spp, Metarhizium spp, etc.) bacteria (Pseudomonas spp, Azotobacter spp, Azospirillum spp, Streptomyces spp, etc.) spore-forming bacteria (Bacillus spp). Particularly preferred is, according to the invention, the Trichoderma spp. microorganism.

The plant allows also the production of at least one product of said at least one microorganism type. With product of said at least one microorganism type it is meant herein viable spores of the above stated microorganisms, secondary metabolites, proteins and enzymes of these microorganisms. Spore means a type of formation which develops, under unfavorable environmental conditions, within the cytoplasm of some aerobic, anaerobic bacteria and fungi. The bacterial spore under suitable environmental and nutritional conditions can germinate and produce another bacterial cell. The fungal spore (conidium) under suitable environmental and nutritional conditions can germinate and produce another fungal cell.

Secondary metabolites means herein substances produced by viable cells via peculiar metabolic pathways different with respect to the primary ones.

According to the invention, the inoculation of the microorganisms into the reactor occurs by means of an outlet attached to the reactor.

The plant of the invention is a solid-state fermentation plant on organic substrates.

The fermentation is the biochemical process by which organic compounds are destroyed into simpler compounds, with the release of a certain amount of energy used by the microorganisms which carried out the fermentation reactions for their metabolic needs.

Advantageously, is has been observed that solid-state fermentation allows to obtain highly concentrated microbiologic products (microorganisms, metabolites, enzymes) without wastes to be treated. The solid-state fermentation requires the presence of a substrate, on which the microorganisms introduced into the plant carry out metabolic processes.

According to the invention, the reactor, the vacuum pump, the filter and the condenser of the plant of the invention are connected each other.

According to a preferred embodiment, the reactor is connected to the filter, and the filter is connected to the condenser in turn connected to the vacuum pump.

The reactor of the plant accommodates the process steps of the invention, including the steps of fermentation of the substrates by the microorganisms and of drying the microorganisms themselves.

In the reactor of the plant the material of the reaction comprising at least one microorganism type according to the process of the invention is inoculated.

Microorganism type means herein small unicellular or multicellular viable organisms, prokaryotes or eukaryotes, belonging to different kingdoms and domains.

Such microorganisms include bacteria, yeasts and fungi.

The term "microorganism" comprises non-genetically modified microorganisms and genetically modified microorganisms.

Such reactor is equipped with an aerator and with a stirring system suitable to avoid the aggregation of the reaction material. According to a preferred embodiment, said stirring system is a screw mixer.

According to the invention, a jacket, used to thermostat the plant, is connected to the reactor. According to the invention, the plant for the growth of microorganisms is equipped with an absolute filter in contact with the reactor by means of an aerator nozzle placed on the bottom of said reactor.

Such absolute filter is suitable to the purification and sterilization of the incoming air necessary for the fermentation and injected by means of said aerator nozzle.

According to a preferred embodiment, the plant of the invention according to any of the embodiments thereof can also comprise means which allow the sampling, i.e. the collection of samples comprising the kind of microorganism being produced, in order to control the progress of the reaction and the quali-quantitative characteristics of the reaction product. Said means which allow the sampling also allow therefore the carrying out of quality controls relating to the sterility of the sample and can be carried out by means of the sterile sampling point connected to the reactor of the plant.

The plant of the invention according to any one of the embodiments thereof, can further comprise one or more sensors allowing the measurement of the physical conditions within the reactor of the plant. According to a preferred embodiment, said sensors are probes. Still according to a preferred embodiment, said probes allow to measure pressure, humidity, CO₂ and temperature. Therefore, the plant of the invention has one or more pressure probes, one or more humidity probes, one or more CO₂ probes and one or more temperature probes attached to the reactor of the plant.

According to a more preferred embodiment of the invention, the probe for the measurement of pressure and the probe for the measurement of temperature are attached to the reactor.

Still according to a more preferred embodiment of the invention, the probe for the measurement of CO₂ is attached to the filter of the plant and the probe for the measurement of humidity is placed between the condenser and the filter of the plant.

The pressure probe has the double function of regulating the air inflow during the fermentation step and of setting up the equilibrium state under drying conditions.

The temperature probe allows to regulate the steam during the sterilization step, allows to keep a constant temperature during the fermentation step and finally allows to control the drying step.

The CO₂ probe has the double function of: allowing to know the condition of the fermentation process and takes part in the regulation of the incoming air flux.

According to a preferred embodiment of the invention, the plant is automatized by a programmable logic controller provided with software. The programmable logic controller acquires the pressure, humidity, CO₂ and temperature conditions from said one or more probes connected to the reactor of the plant and controls the regulation devices. The reactor of the plant can have proximity switches. Proximity switch means herein a type of sensor capable of interrupting the machine operation in case of accidental opening of a reactor inlet.

The reactor of the plant of the invention is equipped with a foot valve to discharge the substrate, once dried.

According to the invention, the vacuum pump creates a vacuum circuit, a novelty element of the present invention. As a matter of fact, such vacuum pump allows to carry out the vacuum drying in the same reactor in which the fermentation and namely the production of the microorganism occurred.

Advantageously, it has been observed that the plant of the invention, based on the mixing and vacuum drying within the same reactor, allows to produce the desired microorganisms under sterility and in short times.

Still more advantageously, said plant allows to produce high yields of the microorganisms of interest.

One of the main advantages of the plant according to the invention is indeed to provide the possibility of carrying out the drying of the microorganisms produced following the fermentation step, which leads to the production of the same microorganisms. In the plants according to the known art, indeed, the fermentation step which leads to the production of the desired microorganisms is ended with the recovery of the microorganism, which must be removed from the reactor and brought in a different place for its drying. This way, in addition to an inevitable reduction of reaction yields, due to the impossibility of recovering 100% of the microorganism produced during the fermentation step, there is a high risk of contamination of the produced microorganism by external agents, since the recovering step of the microorganism from the reactor and the next step of placing the same into the drying equipment involve necessarily, even using all the necessary care and precautions, an exposure of the microorganism to external agents, which is not always of simple control. In the plant according to the present invention, instead, there is no need of recovering the fermentation product and namely the desired microorganism, before its drying, which can instead occur directly within the reactor, thus avoiding the handling of the fermentation product and its consequent exposure to contamination risk. Moreover, this aspect allows to avoid a yield reduction, with respect to the yields of the fermentation itself, proving to be clearly a high cost-performance process on the whole.

The further component of the plant of the invention is the filter. According to a preferred embodiment, such filter is connected to the reactor of the plant and to the condenser and it is necessary for the capture of possible particles directed towards the vacuum pump.

Moreover, the plant of the invention has the condenser connected to the vacuum pump and the filter. According to a preferred embodiment, the condenser is a shell-and-tube condenser. Said condenser has the purpose of condensing the vapors emitted from the product during the drying step, according to the process of the invention. Glycol water cooled by a suitable refrigerator circulates in the condenser of the plant according to the invention.

In addition, the plant of the invention is further equipped with a switchboard for the power supply of the utilities.

The plant of the invention has a washing system. Such washing system is activated at the end of the fermentation, drying and discharge steps of the substrate and the produced microorganisms and has the purpose of cleaning the reactor of the plant, where these steps of the process of the invention have been carried out.

Figure 1 is a schematic representation of an implementation of the plant 100 according to the invention.

The plant 100 comprises a reactor 1, a vacuum pump 6, a filter 7 and a condenser 8. The reactor 1 is connected to the filter 7. The filter 7 is connected to the condenser 8, in turn connected to the vacuum pump 6. The reactor 1 has a jacket 11. The reactor 1 is equipped with a stirring system 9 and an aerator 10. The probe 2 for the measurement of pressure and the probe 5 for the measurement of temperature are attached to the reactor 1. The probe 4 for the measurement of CO₂ is attached to the filter 7. The probe 3 for the measurement of humidity is placed between the condenser 8 and the filter 7. The plant 100 is equipped with and absolute filter 12 placed on the incoming air circuit 121 and bound to the aerator 10 placed within the reactor 1. The reactor 1 has a sterile sampling point 13 attached to it. The outlet 14 is connected to the reactor 1. The reactor 1 is equipped with a foot valve 15 for the product discharge. The plant 100 is equipped with a washing system 16 attached to the reactor 1 and to the probe 2 for the measurement of humidity.

The plant according to any one of the embodiment thereof is suitable for the microorganism growth.

Therefore, further object of the present invention is a process for the growth of at least one microorganism type on a suitable substrate.

The process of the invention is a solid-state fermentation process on organic substrates. Solid-state fermentation on organic substrates means the fermentation process prepared by using solid feedstock acting as substrates, under conditions of absence or limited free water. Therefore, upstream of the process of the invention it is necessary to prepare the pre-weighed dried substrate and to prepare the fermentation medium for the microorganisms which will be inoculated into the plant.

Therefore, upstream of the process of the invention it is necessary to carry out the following steps:
I. Loading the pre-weighed dried substrate into reactor 1 of the plant of the invention
II. Preparing the fermentation medium for the at least one microorganism type
III. Selecting the microorganism/target parameters by the programmable logic controller of the plant of the invention

In step I of the process of the invention organic substrates, preferably solid organic substrates, still more preferably dried organic substrates, can be used. Examples of the substrate which can be used are vegetable materials such as corn, corncob, wheat, flour, seeds, starches, rice, of different particle size. The selection of the substrate or substrate mixture depends in any case on the type and characteristics of the microorganism to be produced. Therefore, the process of the invention comprises the selection of the substrate suitable for the growth of the at least one microorganism type of interest. Such organic substrates are weighed and loaded into the reactor of the plant and will be used by the microorganisms inoculated into the reactor for their growth.

After loading the pre-weighed dried substrate it is necessary to prepare the fermentation medium (step II) to be used in the process of the invention. The components of the fermentation medium, the amounts of said components and the water content to be added depend on the type of microorganism used in the process of the invention.

For example, according to the present invention, a suitable fermentation medium composition is the following:
- Vegetable substrate: 62%
- Water: 25%
- Mannitol: 0.6%
- MgSO₄: 0.25%
- KNO₃: 0.3%
- FeSO₄: 0.15%
- Liquid microbial culture: 11.7%

The components of the fermentation medium provide the nutrients necessary for the microorganism growth.

Step III of microorganism/target parameter selection can be done by the programmable logic controller of the plant of the invention. The microorganism/target parameters started by the programmable logic controller of the plant of the invention are the physical parameters of the process steps of the invention. For example, the physical parameters varying in the process steps of the invention depending on the microorganism used can be temperature, air flow rate, substrate humidity, shaft rotation speed.

Advantageously, the process of the invention allows to select the suitable parameters according to the type of microorganism used.

The selection of the programs is carried out, e.g., by industrial PC panel. Such industrial PC panel is connected to a programmable logic controller provided with software. The industrial PC panel allows to select the process parameters, such as for example physical parameters such as temperature and pressure, depending on the microorganism to be used in the process of the invention. Therefore, the plant of the invention allows to change the physical parameters of the process steps of the invention.

Following the selection of the microorganism/target parameters by the programmable logic controller of the plant of the invention, the process of the invention starts.

The process for the growth of at least one microorganism type on a suitable substrate comprises the steps of:
a) Sterilizing the reactor of the plant of the invention
b) Cooling the reactor of the plant of the invention
c) Inoculating the reactor of the plant of the invention with at least one microorganism type
d) Carrying out the fermentation reaction in the reactor of the plant of the invention, thus obtaining a fermentation product
e) Carrying out vacuum drying of the substrate
f) Discharging the substrate
g) Carrying out the washing of the reactor of the plant of the invention

According to the invention, steps I, II and III described above are carried out upstream of step a) of the process of the invention.

The process of the invention can be an automatized process and controlled by the programmable logic controller provided with software.

According to the process of the invention, in step a) of sterilization, steam is circulated in the jacket connected to the reactor of the plant of the invention described above and through the absolute filter of such plant. The sterilization step is necessary to remove all the possibly present microorganisms, which could enter in competition with the microorganisms inoculated in step c) of the process of the invention.

In step b) the reactor of the plant of the invention is cooled. According to the process of the invention, in such phase water is circulated in the jacket connected to the reactor of the plant of the invention and air is passed through the absolute filter at the inlet of the reactor until reaching the preset temperature. For example, temperatures that can be used in this step of the process are between about 120°C (particularly preferred is the temperature of 121°C) during the sterilization and about 25-28°C during the fermentation.

In step c) at least one microorganism type is inoculated within the reactor. In step c) of the process of the invention, a preset amount of at least one viable microorganism culture is inoculated into the reactor of the plant. The inoculated microbial culture is a liquid microbial culture which can for example be 10-15% of the total composition of the medium. According to the process of the invention, such microorganisms are introduced into the reactor of the plant through the outlet in contact with the reactor of the plant.

According to a preferred embodiment, the process for the growth of at least one microorganism type is characterized in that said at least one microorganism type is selected from fungi, bacteria, spore-forming bacteria, with particular preference to fungi, according to the invention.

Step d) of fermentation occurs immediately after the microorganism inoculation. During this step, thermostated water is circulated in the jacket attached to the reactor of the plant. At the same time, air is entered from the sterilizing filter through the aerator nozzle acting to purify and sterilize the air required for the fermentation. The regulation of the amount of incoming air is due to the PLC, which receives the data of pressure in the chamber from the pressure gauge and of the amount of CO2 detected by the probe installed on the off gas line and manages the opening and closing of the electrovalve of the incoming air line. The flow rate can vary for example from 0.2 L/Kg/min to 0.8 L/Kg/min.

According to the invention, step d) of fermentation occurs in the reactor of the plant of the invention.

During the fermentation step the metabolic processes, by the inoculated microorganisms, occur in step c) of the process. Such metabolic processes can be processes of aerobic (in presence of oxygen) and/or anaerobic (in absence of oxygen) fermentation. Examples of anaerobic fermentation processes are alcoholic fermentation, lactic acid fermentation, propionic acid fermentation, butanediol fermentation, butyric and isopropyl alcohol fermentation.

During the fermentation step, it is possible to perform sterility corrections to humidity or to the nutrients of the solid substrate through the inoculum outlet attached to the reactor of the plant.

A further preferred embodiment of the process of the present invention comprises at the end of the fermentation step a step of collecting, i.e. sampling, intermediate samples comprising at least one microorganism type. The sampling allows to perform a quality control of the sterility and stability conditions of the microbial culture by means of intermediate samplings. Said samplings can be carried out through the sterile sampling point.

The end of fermentation is defined based on the end of the metabolic processes monitored by means of the probe for the analysis of CO₂.

Object of the invention, according to one of the aspects thereof, is a process for the growth of at least one microorganism type, as defined above, and the following recovery of said microorganisms.

At the end of the fermentation step, it is necessary to treat the still wet final products in order to guarantee their stability. According to the invention, such treatment is carried out by vacuum drying. This way, at least one microorganism type is separated from the liquid medium. Therefore, according to the process of the invention, at the end of the fermentation step, step e) of drying the substrate is started. Drying step e) occurs within the reactor of the plant of the invention. Such step e) of substrate drying is carried out under vacuum. Such vacuum is created by the vacuum pump of the plant of the invention described above. In this step, a recirculation of glycol water at the pipe coil of the condenser connected to the vacuum pump and to the filter is started. At the same time, the jacket attached to the reactor of the plant of the invention is thermostated with hot water.

In an advantageous way, step e) of vacuum drying allows to obtain the at least one microorganism type as final result. In step e) of drying, such microorganisms are stabilized on the substrate with is subjected to drying. In this process, the substrate is not accumulated in the time unit and part of it is used by the microorganisms for their growth. The substrate is then dried.

Therefore, the last step of the process of the invention is the discharge of the dried substrate (step f). The discharge of the dried substrate occurs as a function of the humidity value detected by the humidity probe installed at the outlet of the reactor of the plant.

After the discharge of the substrate, the programmable logic controller provided with software starts step g) of washing the reactor of the plant. The reactor washing occurs by the injection of sanitizing solutions at high pressure and high temperature for the preset time. As it will be shown in the experimental section, the use of the plant of the invention for the solid-state fermentation on organic substrates according to the described process allows to obtain high concentrations, e.g. >10⁹ U.f.c/g of microorganisms in short times, e.g. in a period of time from 7 to 10 days, with respect to about 30 days according to known techniques.

Advantageously, the process of the invention allows to obtain microorganisms in short times with respect to the other known processes. As a matter of fact, the drying step of the microorganisms and of the culture medium is automatized and does not require to be carried out by using external equipment known for the purification of cells and/or proteins. This allows to obtain high concentrations of microorganisms in short times. Advantageously, further equipment for the microorganism drying not being necessary, the process of the invention allows to obtain low cost industrial amounts of desired microorganisms. Therefore, further equipment for the separation of the microorganisms not being necessary, the process of the invention allows to save resources with respect to the techniques conventionally used for the microorganism production.

### EXPERIMENTAL SECTION

### Solid-state fermentation of Tricoderma sp. to obtain viable and stable conidia Medium composition w/w 30 kg tot

- Vegetable substrate - corncob: 62%
- Water: 25%
- Mannitol: 0.6%
- MgSO₄: 0.25%
- KNO₃: 0.3%
- FeSO₄: 0.15%
- Liquid microbial culture: 11.7%

### Procedures and operating parameters

### 1. Loading of the fermentor

- The required feedstock have been weighed and loaded into the fermentor

| **Parameters** | **Values** |
|---|---|
| T (°C) | free |
| CO₂(%) | 0 |
| Rotation (%) | 0 |
| P (bar) | 0 |
| Air flow (L/Kg/min) | 0 |
| Time (min.) | 0 |

### 2. Heating A

- From room T (about 25 °C) to 99 °C
- Steam Injected into the jacket, steam to the inoculum outlet, steam to steam-cross sampling point

| **Parameters** | **Values** |
|---|---|
| T (°C) | Current-99 |
| CO₂ (%) | free |
| Rotation | 40 rpm |
| P (bar) | 0.8 max |
| Air flow (L/Kg/min) | 0 |
| Time (min.) | free |

### 3. Heating B

- From 99.9°C to sterilization temperature equal to 121°C.
- Steam injected into the jacket, steam to the inoculum outlet, steam to the filter and from the filter to the sparger, steam to steam-cross sampling point

| **Parameters** | **Values** |
|---|---|
| T (°C) | **121** |
| CO₂ (%) | Free |
| Rotation | 40 rpm |
| P (bar) | **1.1** |
| Air flow (L/Kg/min) | 0 |
| Time (min.) | 0 |

### 4. Sterilization:

- Sterilization temperature (T): 121°C
- Steam injected into the jacket, steam-cross sampling point, air filter and from this to the sparger, to the inoculum outlet

| **Parameters** | **Values** |
|---|---|
| T (°C) | **121** |
| CO₂ (%) | free |
| Rotation | 60 rpm |
| P (bar) | **1.1°C** |
| Flow (L/Kg/min) | 0 |
| Time (min.) | **30** |

### 5. Cooling up to T 30°C (and, afterwards, to the working T).

- At about 105°C, sterile air has been injected into the chamber to maintain a positive P and to cool. Flow rate: from 0 (initial) to 0.8 L/Kg/min
- Water in the jacket: Max
- Sampling from sampling point and performing the sterility controls

| **Parameters** | **Values** |
|---|---|
| T (°C) | 30 |
| CO₂ (%) | free |
| Rotation | 100 rpm |
| P (bar) | (0.8)/**1.1-0.3** |
| Air flow (L/Kg/min) | 0.8 |
| Time (min.) | free |

### 6. Operations after the confirmation of sterility from the laboratory.

- After the confirmation of the medium sterility has been received, the inoculation under sterility of the liquid microbial culture, according to the preset amount, has been carried out

| **Parameters** | **Values** |
|---|---|
| T (°C) | 30 |
| CO₂ (%) | free |
| Rotation | 60 rpm |
| P (bar) | 0.3 |
| Air flow (L/Kg/min) | 0.1 |
| Time (min.) | free |

### 6-bis. CO₂ calibration min

- Air flow set equal to 0.5 L/Kg/ min and pressure to **0.3 bar.** Wait for the increase of the CO₂ value up to the maximum value, afterwards such value as 0% of saturation has been set.

| **Parameters** | **Values** |
|---|---|
| T (°C) | 28 |
| CO₂ (%) | 0.04% |
| Rotation | 60 rpm |
| P (bar) | 0.3 |
| Air flow (L/Kg/min) | 0.5 |
| Time (min.) | 10 min |

### 7. Growth Start

- Duration of the inoculum up to 4% CO₂ increase

| **Parameters** | **Values** |
|---|---|
| T (°C) | 28 |
| CO₂ max(%) | 0-4 |
| Rotation | Intermittent, 5 min per hour. Speed 30 rpm |
| P (bar) | 0.3 - 0.4 |
| Air flow (L/Kg/min) | 0.3 - 0.7 |
| Time (min.) | 1500 |

### 8. Growth and spore-formation

- The incoming air flow rate has been regulated as a function of the pressure and CO₂ levels recorded

| **Parameters** | **Values** |
|---|---|
| T (°C) | 28 |
| optimal CO₂ (%) | 1 |
| Rotation | Intermittent. Intermittent, 5 min/2 hours. Speed 30 rpm |
| P (bar) | 0.3 - 0.5 |
| Air flow (L/Kg/min) | 0.3 - 0.7 |
| Time (min.) | 3000 |

### 9. Drying

- A sampling has been performed before going on, in order to determine if the fermentation and the spore-formation would be ended.

| **Parameters** | **Values** |
|---|---|
| T(°C) | 35 |
| CO₂ max(%) | 0 |
| Rotation | 100 rpm |
| P (bar) | 0.05 |
| Air flow (L/h) | 0 |
| Time (min.) | 1500 |

### Results

- Conidia concentration: 1x10⁹ ufc/g
- Final water content: 6% w/w
- Active water: 0.4

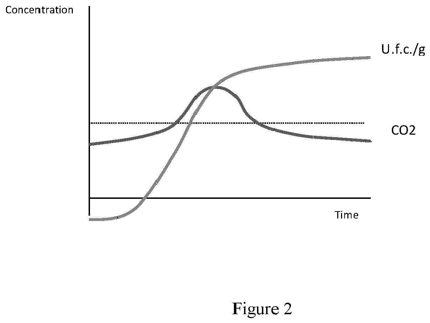

Figure 2 shows the ideal trend of growth and CO₂ production relative to the solid-state fermentation of Tricoderma sp. to obtain viable and stable conidia, as stated in the above indicated experimental section.

## Claims

1. A plant (100) for the growth and production of at least one microorganism type, providing for the growth of said microorganism and the vacuum drying of said microorganism and comprising:
- Reactor (1)
- Filter (7)
- Condenser (8)
- Vacuum pump (6)
**characterized in that** said drying is carried out in the reactor (1) at the end of said microorganism growth.

2. The plant (100) according to claim 1, **characterized in that** it is a solid-state fermentation plant on a substrate.

3. The plant (100) according to claim 1, **characterized in that** it is automatized by a programmable logic controller provided with software.

4. A process for the growth of at least one microorganism type on suitable substrate, comprising the steps of:
a) Sterilizing the reactor (1) of the plant (100) of claim 1
b) Cooling the reactor (1) of the plant (100) of claim 1
c) Inoculating the reactor (1) of the plant (100) of claim 1 with at least one microorganism type
d) Carrying out the fermentation reaction in the reactor (1) of the plant (100) of claim 1, thus obtaining a fermentation product
e) Carrying out vacuum drying of the substrate in the reactor (1) of the plant (100) of claim 1
f) Discharging the substrate
g) Carrying out the washing of the reactor (1) of the plant (100) of claim 1.

5. A process according to claim 4 comprising, downstream of step d), the further step of taking intermediate samples comprising at least one microorganism type.

6. The process according to claim 4 comprising, upstream of step a), the further steps of:
I. Loading the pre-weighed dried substrate into the reactor 1 of the plant of claim 1
II. Preparing the fermentation medium for the at least one microorganism type of claim 1
III. Selecting the microorganism/target parameters by the programmable logic controller of the plant (100) of claim 1.

7. The process according to claim 4, **characterized in that** the substrate of step a) is an organic substrate selected from corn, corncob, wheat, flour, seeds, starches, rice.

8. The process according to claim 4, **characterized in that** said at least one microorganism type is selected from: basidiomycetes fungi, bacteria, sporogenic and actinomycetes bacteria.

9. The process according to claim 8, **characterized in that** said at least one microorganism type is selected from: Trichoderma spp, Metarhizium spp, Pseudomonas spp, Azotobacter spp, Azospirillum spp, Streptomyces spp, Bacillus spp.

10. Use of the plant according to claim 1 for the solid-state fermentation on organic substrates, obtaining microorganisms.
